# FASCICULE DE BREVET EUROPEEN

(11) **EP 0 478 977 B1**
(45) Date de publication et mention de la délivrance du brevet: **18.12.1996**
(21) Numéro de dépôt: 91114991.2
(22) Date de dépôt: 05.09.1991
(51) Int. Cl.: C07C 11/21, C07C 1/32, C07C 1/30, C07C 211/23, C07B 53/00

(54) **Procédé pour la préparation d'oléfines polyinsaturées**
Verfahren zur Herstellung von mehrfach ungesättigten Olefinen
Process for the preparation of polyunsaturated olefins

(30) Priorité: 03.10.1990 CH 3181/90
(43) Date de publication de la demande: 08.04.1992
(73) Titulaire: FIRMENICH SA, CH-1211 Genève 8 (CH)
(72) Inventeur: Chapuis, Christian, CH-1295 Mies (CH)
(74) Mandataire: Salvaterra-Garcia, Maria de Lurdes

(56) Documents cités:
- EP-A- 0 203 615
- DE-A- 1 668 681
- FR-A- 2 309 498
- US-A- 4 467 118

## Description

La présente invention a trait au domaine de l'industrie de la parfumerie. En particulier, elle a pour objet un procédé pour la préparation d'un composé oléfinique polyinsaturé, l'undéca-1,3,5-triène, ingrédient parfumant fort apprécié.

Depuis sa découvert [voir: Chrétien-Bessière et al., Bull. Soc. Chim. Fr. 1967, 97], nombreuses ont été les voies de synthèse proposées pour sa préparation [voir par exemple: brevet FR 74 19580; brevet AS 68 01077; brevet FR 2 309 498 demande de brevet EP 203 615; F. Naef et al., Helv. Chim. Acta 1975, 58, 1016; V. Ratovelomanana et al.; Bull. Soc. Chim. Fr 1987, 174; Recherches 1967, 16, 5; W. Boland et al., Helv. Chim. Acta 1987, 70, 1025; E. Block et al., J. Amer. Chem. Soc. 1986, 108, 4568]. Certaines des méthodes proposées ont trouvé une application industrielle et l'undéca-1,3,5-triène est à présent commercialisé sous des dénominations diverses. Caractérisé par la présence dans sa molécule de trois doubles liaisons éthyléniques, l'undéca-1,3,5-triène peut se présenter sous différentes formes isomériques dont les proportions respectives dans le produit final déterminent sa qualité olfactive. Or, nous avons pu constater qu'aucun des procédés décrits dans l'art antérieur pouvait tout à la fois satisfaire aux exigences d'économie, de sécurité ou de respect de l'environnement et offrir un produit de qualité irréprochable, supérieure à celle du produit présentement offert sur le marché.

Le procédé de l'invention présente l'avantage de permettre l'obtention d'undéca-1,3,5-triène sous sa forme isomérique d'undéca1,3E,5Z-triène pratiquement pure. Or c'est précisément cet isomère qui développe au mieux les propriétés odorantes les plus caractéristiques et prisées parmi l'ensemble des stéréoisomères possibles.

La présente invention apporte donc une solution originale au problème posé par l'obtention de cette spécialité. L'invention a pour objet en particulier un procédé dont l'étape clé consiste en la préparation de composés acétyléniques de formule

H₁₁C₅-C≡C-CH₂-C≡C-CH₂X (I)

dans laquelle X sert à désigner un groupe hydroxyle ou - NR₂, R étant un reste alkyle inférieur, ou un groupe -N-pipéridine ou -N-morpholine.

Parmi les composés de formule (I), il convient de citer les composés aminés (X= - NR₂) dont la structure est nouvelle, composés par ailleurs d'emploi préférentiel selon un mode d'exécution du procédé de l'invention.

Le procédé de l'invention repose sur une synthèse sélective basée sur des étapes de réaction classique mais dont le choix en vue de la préparation d'undécatriène n'avait jamais été mentionné ni même suggéré jusqu'ici. De telles réactions obéissent aux critères de haute sélectivité requis pour l'obtention de l'isomère préférentiel avec de bons rendements et sans avoir à recourir à des étapes complexes de purification.

Le procédé de l'invention consiste en les étapes consécutives suivantes :
a. conversion du déca-1,4-diyne en une undéca-2,5-diynyl-amine N,N-disubstituée par traitement dudit déca-1,4-diyne avec du formaldéhyde et une amine secondaire dans les conditions d'une réaction de type Mannich, suivie
b. d'une réduction du composé ainsi obtenu au moyen d'une hydrogénation catalytique pour donner une undéca-2Z,5Z-diényl-amine N,N-disubstituée, et
c. d'une réaction d'élimination, dans les conditions d'une réaction de type dit d'Hoffmann, consistant en la quaternarisation de la undéca-2Z,5Z-diényl-amine N,N-disubstituée obtenue, transformation en l'hydroxyde d'ammonium correspondant et décomposition; ou
d. conversion de la undéca-2Z,5Z-diényl-amine N,N-disubstituée en N-oxyde correspondant, et
e. traitement thermique du N-oxyde obtenu, pour fournir l'undéca-1,3E,5Z-triène désiré.

Le procédé défini plus haut peut être illustré par le schéma réactionnel suivant :

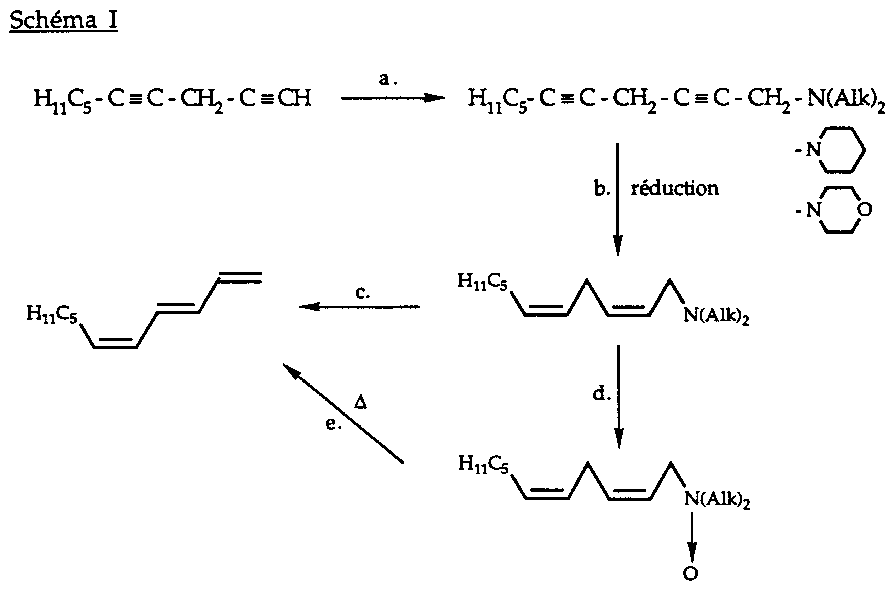

La déca-1,4-diyne, produit de départ dans le procédé de l'invention, est un composé de structure connue qui peut être obtenu conformément à un procédé décrit dans la littérature [L. Brandsma, Preparative Acetylenic Chemistry, exp. 34, p. 52, Elsevier (1971)] au moyen d'une réaction de Grignard entre le 1-heptyne et le bromure d'éthyl-magnésium suivie de l'addition de bromure de propargyle. D'autre part, ce même déca-1,4-diyne peut être synthétisé, conformément à la méthode décrite dans Synthesis 1979, 292, à partir du p-toluènesulfonate de propargyle, lequel composé est obtenu aisément à partir de l'alcool propargylique [voir la méthode suggérée par L. Brandsma, op. cit., exp. 5, p. 159].

Le déca-1,4-diyne ainsi obtenu est maintenu de préférence en solution dans un solvant inerte approprié, tel un éther comme le tétrahydrofuranne. Il est en effet apparu à l'usage que ce composé acétylénique est instable et qu'il convient, par conséquent, de procéder rapidement à son emploi selon l'invention.

La première étape de la réaction s'effectue selon les conditions usuelles appliquées à une réaction de type de Mannich [voir: Herbert O. House, Modern Synthetic Reactions, 2nd édition, Benjamin In. (1972) : L. Brandsma, op. cit., exp. 44, p. 59]. A titre d'amine secondaire, on peut employer une dialkylamine telle la diéthylamine. L'étape suivante d'hydrogénation est conduite dans un réacteur ordinaire et en présence d'un catalyseur d'hydrogénation usuel connu pour promouvoir la réduction des liaisons acétyléniques en oléfines d'isomérie cis. Un catalyseur au palladium partiellement empoisonné, dit de type de Lindlar, convient parfaitement à ce type de réaction [voir Fieser et Fieser, Reagents for Organic Synthesis, p. 566 (1967)]. Lors de l'hydrogénation, il faut veiller à arrêter la réduction après absorption de 2 équivalents d'hydrogène, ce qui évite la formation de produits provenant d'une hydrogénation ultérieure de l'oléfine formée.

L'élimination de type d'Hoffmann [voir Organic Reactions 1960, 11, 317] qui caractérise l'étape c. du procédé peut s'effectuer, après quaternarisation de l'amine oléfinique obtenue au moyen d'une addition d'un halogénure d'alkyle, par exemple l'iodure de méthyle, par traitement du sel d'ammonium résultant avec le tert-butylate de potassium à température ambiante ou, alternativement, par méthylation et élimination simultanée à l'aide d'un traitement avec du sulfate de diméthyle en présence d'hydroxyde de sodium dans l'eau à reflux.

Selon une variante du procédé de l'invention, la undéca-2Z,5Z-diényl-amine N,N-disubstituée est transformée en son dérivé N-oxyde correspondant puis l'oxyde obtenu est soumis à un traitement thermique pour donner l'undéca-1,3E,5Z-triène désiré. La formation du N-oxyde peut s effectuer par analogie avec la méthode décrite par A.C. Cope et E. Ciganek [Organic Synthesis Coll. vol. IV, Rabjohn, p. 612]. Le traitement thermique suivant a lieu par pyrolyse à une température d'environ 170-190°C. La variante que l'on vient de décrire permet l'obtention de l'undécatriène désiré accompagné par des quantités équivalentes de l'isomère undéca-2Z,4Z,6E-triène, la séparation de ces deux composants pouvant être effectuée par l'une des méthodes courantes de séparation.

La présente invention, outre le procédé défini plus haut, a également pour objet les composés de formule (I) dont le symbole X définit un reste - NR₂, R étant un groupe alkyle inférieur.

L'invention a trait également à un procédé pour l'obtention d'undéca-1,3E,5Z-triène, lequel procédé est caractérisé en ce qu'on
i. réagit le déca-1,4-diyne avec un halomagnésien d'alkyle, puis avec du formaldéhyde pour fournir l'undéca-2,5-diyn-1-ol,
ii. soumet ledit diynol à une hydrogénation catalytique pour donner l'undéca-2Z,5Z-diénol,
iii. transforme le diénol ainsi formé en le chlorure d'undéca-2Z,5Z-diényle, et
iv. soumet ce dernier composé à une réaction d'élimination à l'aide d'un agent basique constitué par un alcoolate d'un métal alcalin.

Le procédé défini ci-dessus est illustré à l'aide du schéma réactionnel que voici :

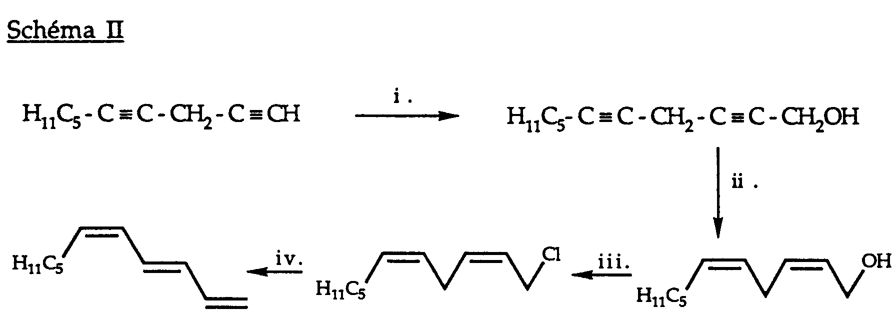

La présente invention sera illustrée à l'aide des exemples suivants dans lesquels les températures sont indiquées en degrés centigrades et les abréviations ont le sens usuel dans l'art.

### Exemple 1

On a préparé du déca-1,4-diyne, utilisé comme produit de départ dans le procédé de l'invention, de la manière suivante.

Dans un ballon de 1 l, on a placé 19,2g de magnésium en copeaux (0,8 mole) dans 550 ml de tétrahydrofuranne (THF) sous atmosphère d'azote. A ce mélange, on a ajouté goutte à goutte de façon à maintenir le THF au reflux 84 g (0,77 mole) de bromure d'éthyle. Une fois le magnésien formé, on a ajouté goutte à goutte à 10° 62,4 g (0,65 mole) de 1-heptyne en 10 min, puis le tout a été porté à 60° pendant 45 min. Après refroidissement à 10°, on a ajouté au mélange 1,93 g (19,5 mmole) de CuCl et on a maintenu au repos pendant 15 min. 138 G (0,65 mole) de p-toluènesulfonate de propargyle ont été ensuite ajoutés en 20 min au mélange réactionnel maintenu à 0°. Puis le tout a été laissé au repos pendant 30 min en veillant à ce que la température ne dépasse pas 5°. Puis elle a été portée à 15° afin de terminer la réaction. On a hydrolysé par adjonction de 100 ml d'eau puis on a séparé et extrait la phase aqueuse avec 2 fractions de 80 ml chacune de THF. Les extraits organiques combinés ont été ensuite lavés avec une solution aqueuse saturée de NaCl, 2 fractions de 50 ml chacune de KCN à 5% dans l'eau et enfin séchés sur Na₂SO₄. Après filtration et évaporation, on a obtenu une solution concentrée du déca-1,4-diyne dans le THF, solution qui a été utilisée pour l'étape suivante de la réaction :
a. On a placé dans un réacteur de 1l 19,5 g (0,65 mole) de paraformaldéhyde, 54,8 g (0,75 mole) de diéthylamine, 2,6 g d'acétate de cuivre (II) hydraté et 65 ml de THF. Ce mélange a été maintenu à reflux pendant 30 min et, toujours à reflux, on y a ajouté la solution de déca-1,4-diyne obtenue comme décrit plus haut. Le mélange de réaction a été maintenu à reflux pendant 1 h 1/2 jusqu'à disparition complète du déca-1,4-diyne. Le mélange a été ensuite extrait à 0° avec une solution à 15% d'HCl (3x70 ml) et les phases acides ont été extraites avec 2 fractions de 50 ml chacune d'éther de pétrole 80:100°, puis 150 ml d'une solution de NaOH à 30% y ont été ajoutés à 0°. La phase basique ainsi obtenue a été extraite avec 3 fractions de 150 ml chacune d'acétate d'éthyle et les extraits organiques traités avec une solution aqueuse saturée de NaCl, séchés sur Na₂SO₄, filtrés et évaporés pour obtenir 105 g de N,N-diéthyl-undéca-2,5-diynyl-amine.
   - IR :: 2940, 1460, 1310, 1200, 1090 cm⁻¹
   - ¹H-RMN:: 0,9(3H,t,J=7) ; 1,07(6H,t,J=7) ; 1,3-1,55(6H,m) ; 2,15(2H,m) ; 2,55(4H,q,J=7) ; 3,15(2H,m) ; 3,40(2H,t,J=2,5) δ ppm
   - SM :: M⁺ = 219(4) ; 204(100), 91(30), 58(21)
b. 105 G de l'amine préparée comme indiqué sous lettre a. en solution dans 500 ml de cyclohexane ont été placés dans un réacteur d'hydrogénation de 1,5 l dans lequel on a ajouté 2,7g de catalyseur de Lindlar et 2 gouttes de quinoline. L'hydrogénation a été effectuée à pression ambiante et elle a été interrompue après absorption de 2 équivalents d'hydrogène. Par filtration, évaporation et distillation dans un four à boules, on a obtenu 99,8 g de N,N-diéthyl-undéca-2Z,5Z-diényl-amine.
   - Rend. :: 95% ; Eb. : 60°/2,66 Pa.
   - IR :: 2950, 1650, 1460, 1380, 1200, 1060 cm⁻¹
   - ¹H-RMN:: 0,9(3H,t,J=7) ; 1,05(6H,t,J=7) ; 1,3(6H,m) ; 2,05(2H,q,J=7) ; 2,53(4H, q,J=7) ; 2,82(2H,t,J=5) ; 3,15(2H,d,J=5) ; 5,37(2H,m) ; 5,5(2H,t,J=5) δ ppm
   - SM :: M⁺ = 223(3) ; 208(4), 164(5), 150(30), 125(27), 112(100), 110(77), 79(86), 58(75)
c. 4,5G (20,18 mmole) de l'amine obtenue sous lettre b. ci-dessus dans 15 ml d'acétate d'éthyle ont été placés dans un ballon de 50 ml sous atmosphère d'azote, puis 4,26 g (30 mmole) d'iodure de méthyle y ont été ajoutés. Après avoir maintenu le mélange 1 h à température ambiante, le solvant a été évaporé sous vide et 10ml de tert-butanol y ont été ajoutés suivis, à 5°, de 3,36 g (30 mmole) de tert-butylate de potassium. Le mélange a été maintenu à température ambiante pendant 20 min, puis on a ajouté 30 ml d'eau et 30 ml d'acétate d'éthyle. Après séparation, la phase aqueuse a été reprise avec 2 fractions de 10 ml chacune d'acétate d'éthyle et les extraits organiques ont été lavés avec une solution aqueuse saturée (10 ml) de NaCl, tandis que le pH a été ajusté à 5-6 par addition de 15 ml d'une solution d'acide oxalique à 10%. Après un nouveau lavage avec 10 ml d'une solution aqueuse saturée de NaCl, 10 ml d'une solution de bicarbonate de sodium et 2 nouvelles fractions de 10 ml chacune de NaCl, les extraits organiques ont été séchés sur Na₂SO₄, filtrés, évaporés et distillés dans un four à boules pour obtenir 2,34 g (15,13 mmole) d'undéca-1,3E,5Z-triène ayant une pureté d'environ 95%.
   - Rend :: 75%.

### Variante

La conversion de la N,N-diéthyl-undéca-2Z,5Z-diéthylamine en undéca-1,3E,5Z-triène peut également être effectuée en procédant de la manière suivante.

3,9 G (17,5 mmole) de l'amine ont été placés dans un ballon de 25 ml avec 2,8 g (70 mmole) de NaOH en solution dans 12 ml d'eau et le mélange obtenu a été porté à ébullition, puis 4,4 g (35 mmole) de sulfate de diméthyle y ont été ajoutés goutte à goutte. Après 1 h, on a ajouté une nouvelle fraction de 1,4g de NaOH (35 mmole) et 2,2 g (17,5 mmole) de sulfate de diméthyle. Après avoir été laissé à reflux pendant 1 h, on a refroidi et extrait avec de l'acétate d'éthyle. En suivant la méthode d'extraction indiquée sous lettre c. ci-dessus, on a obtenu 1,8 g (rend. : 67%) de l'undécatriène désiré dont la pureté était de 95%.

### Exemple 2

### (voir Schéma I, étape d.)

d. 4 G (18 mmole) de N,N-diéthyl-undéca-2Z,5Z-diényl-amine dans 10 ml d'éthanol ont été placés dans un ballon de 25 ml sous atmosphère d'azote, puis 4ml d'eau oxygénée à 70% y ont été ajoutés. Le mélange réactionnel a été maintenu sous agitation pendant 1 h puis il a été laissé au repos à température ambiante pendant 18 h. Après évaporation, il a été repris avec de l'acétate d'éthyle et la phase organique, une fois séparée, a été lavée avec une solution saturée de NaCl, séchée sur Na₂SO₄ et évaporée pour fournir 4,09 g de N,N-diéthyl-undéca-2Z,5Z-diényl-amine N-oxyde (rend. : 95%).
   - IR :: 2940, 1650, 1460, 1380, 780 cm⁻¹
   - ¹H-RMN(80 MHz):: 0,9(3H,t,J=7) ; 1,1(6H,t,J=7) ; 1,3(6H,m) ; 2,04(2H,m) ; 2,75(2H,m) ; 3,33(4H,q,J=7) ; 3,95(2H,d,J=5) ; 5,3(2H,m) ; 5,65(2H,m) δ ppm
   - SM :: M⁺ = 239(1) ; 151(8), 109(18), 95(39), 89(92), 81(50), 74(100), 67(67), 55(49), 41(36)

### (voir Schéma I, étape e.)

e. Le N-oxyde obtenu sous chiffre d. ci-dessus a été distillé dans un four à boules à 180° sur pression ordinaire pour donner 2,48 g (16,5 mmole) d'un mélange 1:1 d'undéca-1,3E,5Z-triène et d'undéca-2Z,4Z,6E-triène.

### Example 3

### (voir Schéma II, étape i.)

i. On a préparé une solution dans le THF de déca-1,4-diyne en suivant la méthode indiquée à l'Exemple 1, puis on a ajouté à 0° un équivalent de bromure d'éthyle magnésium et le tout a été maintenu à 60° pendant 45 min. Après avoir rajouté au mélange 1,2 equivalent de paraformaldéhyde, on a porté le mélange à reflux pendant 3 h. Après refroidissement, on a extrait avec de l'éther de pétrole 80:100° et les phases organiques ont été lavées avec de l'eau puis avec une solution aqueuse saturée de NaCl, puis séchées et évaporées. Le résidu obtenu a été chromatographié sur une colonne remplie de silice en utilisant comme éluant un mélange de 1 à 10% d'acétate d'éthyle dans le cyclohexane. On a ainsi obtenu de l'undéca-2,5-diyn-1-ol avec un rendement de 40%.
   - IR :: 3350, 2940, 2200, 1460, 1320, 1010 cm⁻¹
   - ¹H-RMN :: 0,9(3H,t,J=7) ; 1,25-1,55(6H,m) ; 2,15(2H,m) ; 3,20(2H,t, J=2,5) ; 4,26(2H,t,J=2,5) δ ppm
   - SM :: M⁺ = 164(1) ; 131(17), 115(23), 105(29), 95(86), 91(97), 79(100), 77(89), 67(52), 55(42), 41(61)

### (voir Schéma II, étape ii.)

ii. 6,2 G (37,8 mmole) du carbinol obtenu comme indiqué sous lettre i. ci-dessus dissous dans 50 ml de cyclohexane ont été hydrogénés à pression ambiante en présence de 120 mg de catalyseur au palladium de type Lindlar. Après filtration et évaporation, on a chromatographié le résidu obtenu sur une colonne de silice en utilisant comme éluant un mélange de 1 à 10% d'acétate d'éthyle dans le cyclohexane pour fournir 5,9 g (rend. : 95%) d'undéca-2Z,5Z-diénol.
   - IR :: 3350, 2930, 1460, 1040 cm⁻¹
   - ¹H-RMN :: 0,9(3H,t,J=7) ; 1,3(6H,m) ; 2,05(2H,q,J=7) ; 2,83(2H,t,J=7) ; 4,22(2H,d,J=7); 5,3-5,45(2H,m) ; 5,5-5,65(2H,m) δ ppm
   - SM :: M⁺ = 168(1) ; 150(10), 91(30), 79(100), 67(57), 55(50), 41(63)

### (voir Schéma II, étape iii.)

iii. 4,8 G (28,6 mmole) de l'alcool oléfinique obtenu sous lettre ii. ci-dessus ont été maintenus à reflux pendant 1 h dans 80 ml de CCl₄ en présence de 11,3 g (43 mmole) de triphénylphosphine. Après évaporation du solvant, le résidu a été filtré sur une colonne remplie de silice avec comme éluant un mélange de 1 à 5% d'acétate d'éthyle dans le cyclohexane pour fournir 4,16 g de chlorure d'undéca-2Z,5Z-diényle (rend. : 78%).
   - IR :: 2940, 1460 cm⁻¹
   - ¹H-RMN(80MHz) :: 0,9(3H,t,J=7) ; 1,3(6H,m) ; 2,05(2H,q,J=7) ; 2,88(2H,t, J=7) ; 4,13(2H,d,J=7) ; 5,33(1H,m) ; 5,44(1H,m); 5,63(2H,m) δ ppm
   - ¹³C-RMN :: 15,37(q) ; 23,9(t) ; 26,9(t) ; 28,6(t) ; 30,6(t) ; 32,9(t) ; 40,65(t) ; 126,7(d) ; 127,6(t) ; 132,9(d) ; 135,0(d) δ ppm
   - SM :: M⁺ = 186(1) ; 150(8), 116(10), 102(32), 91(30), 79(68), 67(100), 55(47), 41(28)

### (voir Schéma II, étape iv.)

iv. Le chlorure d'undéca-2Z,5Z-diényle brut tel qu'obtenu comme indiqué ci-dessus a été dilué dans 15ml de tert-butanol et additionné de 3,36 g (30 mmole) de tert-butylate de potassium à 0°. Après avoir agité le mélange pendant 1 h, on a extrait et traité comme indiqué à l'Exemple 1, lettre c. pour fournir, avec un rendement de 72%, un mélange contenant, à raison de 95%, de l'undéca-1,3E,5Z-triène accompagné de 4% d'undéca-1,3Z,5E-triène.

## Revendications

1. Procédé pour la préparation d'undéca-1,3E,5Z-triène, caractérisé par les étapes consécutives suivantes :
a. conversion du déca-1,4-diyne en une undéca-2,5-diynyl-amine N,N-disubstituée par traitement dudit déca-1,4-diyne avec du formaldéhyde et une amine secondaire dans les conditions d'une réaction de type Mannich, suivie
b. d'une réduction du composé ainsi obtenu au moyen d'une hydrogénation catalytique pour donner une undéca-2Z,5Z-diényl-amine N,N-disubstituée, et
c. d'une réaction d'élimination, dans les conditions d'une réaction de type dit d'Hoffmann, consistant en la quaternarisation de la undéca-2Z,5Z-diényl-amine N,N-disubstituée obtenue, transformation en l'hydroxyde d'ammonium correspondant et décomposition ; ou
d. conversion de la undéca-2Z,5Z-diényl-amine N,N-disubstituée en N-oxyde correspondant, et
e. traitement thermique du N-oxyde obtenu, pour fournir l'undéca-1,3E,5Z-triène désiré.

2. Procédé selon la revendication 1, caractérisé en ce qu'on utilise à titre d'amine secondaire la diéthylamine et que l'on obtient, suivant l'étape a., la N,N-diéthyl-undéca-2,5-diényl-amine.

3. Procédé selon la revendication 1, caractérisé en ce que l'hydrogénation catalytique, suivant l'étape b., s'effectue en présence d'un catalyseur au palladium partiellement empoisonné.

4. Procédé selon la revendication 1, caractérisé en ce que la quaternarisation de la undéca-2Z,5Z-diényl-amine N,N-disubstituée s'effectue par addition d'un halogénure d'alkyle suivie d'un traitement avec le tert-butylate de potassium ou par méthylation et élimination simultanée avec du sulfate de diméthyle en présence d'hydroxyde de sodium à reflux.

5. Une undéca-2,5-diynyl-amine N,N-disubstituée de formule
H₁₁C₅-C≡C-CH₂-C≡C-CH₂NR₂
dans laquelle R représente un reste alkyle inférieur.

6. N,N-Diéthyl-undéca-2,5-diynyl-amine

7. Procédé pour la préparation d'undéca-1,3E,5Z-triène caractérisé en ce qu'on :
i. réagit le déca-1,4-diyne avec un halomagnésien d'alkyle, puis avec du formaldéhyde pour fournir l'undéca-2,5-diyn-1-ol,
ii. soumet ledit diynol à une hydrogénation catalytique pour donner l'undéca-2Z,5Z-diénol,
iii. transforme le diénol ainsi formé en le chlorure d'undéca-2Z,5Z-diényle, et
iv. soumet ce dernier composé à une réaction d'élimination à l'aide d'un agent basique constitué par un alcoolate d'un métal alcalin.

8. Procédé selon la revendication 7, caractérisé en ce qu'on utilise, comme alcoolate d'un métal alcalin, le tert-butylate de potassium.

## Claims

1. Process for the preparation of undeca-1,3E,5Z-triene, characterized by the following consecutive steps:
a. conversion of deca-1,4-diyne into a N,N-disubstituted undeca-2,5-diynyl-amine by way of treatment of said deca-1,4-diyne with formaldehyde and a secondary amine under the conditions of a Mannich type reaction; followed by
b. reduction of the thus obtained compound by means of a catalytic hydrogenation to give a N,N-disubstituted undeca-2Z,5Z-dienyl-amine; and
c. an elimination reaction, under the conditions of a so-called Hoffmann type reaction, consisting in the quaternarization of the obtained N,N-disubstituted undeca-2Z,5Z-dienyl-amine, conversion into the corresponding ammonium hydroxide and decomposition; or
d. conversion of said N,N-disubstituted undeca-2Z,5Z-dienyl-amine into the corresponding N-oxide; and
e. thermal treatment of the obtained N-oxide to give the desired undeca-1,3E,5Z-triene.

2. Process according to claim 1, characterized in that diethylamine is used as the secondary amine and N,N-diethyl-undeca-2,5-dienyl-amine is obtained according to reaction step a.

3. Process according to claim 1, characterized in that the catalytic hydrogenation according to step b. is carried out in the presence of a partially poisoned palladium catalyst.

4. Process according to claim 1, characterized in that the quaternarization of said N,N-disubstituted undeca-2Z,5Z-dienyl-amine is carried out by addition of an alkyl halide, followed by a treatment with potassium tert-butylate, or by methylation and simultaneous elimination with dimethyl sulfate in the presence of sodium hydroxide at reflux.

5. A N,N-disubstituted undeca-2,5-diynyl-amine of formula
H₁₁C₅-C≡C-CH₂-C≡C-CH₂NR₂
wherein R represents a lower alkyl radical.

6. N,N-Diethyl-undeca-2,5-diynyl-amine.

7. Process for the preparation of undeca-1,3E,5Z-triene, characterized in that
i. deca-1,4-diyne is reacted with an alkyl magnesium halide and then formaldehyde to give undeca-2,5-diyn-1-ol;
ii. said diynol is subjected to catalytic hydrogenation to give undeca-2Z,5Z-dienol;
iii. the thus formed dienol is converted into undeca-2Z,5Z-dienyl chloride; and
iv. the latter compound is subjected to an elimination reaction by means of a basic agent consisting of an alkali metal alkoxide.

8. Process according to claim 7, characterized in that potassium tert-butoxide is used as the alkali metal alkoxide.

## Patentansprüche

1. Verfahren zur Herstellung von Undeka-1,3E,5Z-trien, gekennzeichnet durch die folgenden aufeinanderfolgenden Stufen:
a. Umwandlung von Deka-1,4-diin in ein N,N-disubstituiertes Undeka-2,5-diinylamin durch Behandlung des Deka-1,4-diins mit Formaldehyd und einem sekundären Amin unter den Bedingungen einer Reaktion vom Mannich-Typus, gefolgt
b. von einer Reduktion der so erhaltenen Verbindung mittels katalytischer Hydrierung, um ein N,N-disubstituiertes Undeka-2Z,5Z-dienylamin zu ergeben, und
c. von einer Eliminierungsreaktion unter den Bedingungen einer Reaktion vom sogenannten Hoffmann-Typus, bestehend aus der Quatärnisierung des erhaltenen N,N-disubstituierten Undeka-2Z,5Z-dienylamins, Umwandlung in das entsprechende Ammoniumhydroxid und Zersetzung; oder
d. Überführung des N,N-disubstituierten Undeka-2Z,5Z-dienylamins in das entsprechende N-Oxid und
e. thermische Behandlung des erhaltenen N-Oxids, um das gewünschte Undeka-1,3E,5Z-trien zu ergeben.

2. Verfahren gemäss Patentanspruch 1, dadurch gekennzeichnet, dass man als sekundäres Amin Diethylamin verwendet und dass man gemäss Stufe a. N,N-Diethyl-undeka-2,5-dienylamin erhält.

3. Verfahren gemäss Patentanspruch 1, dadurch gekennzeichnet, dass die katalytische Hydrierung gemäss Stufe b. in Anwesenheit eines teilweise vergifteten Palladiumkatalysators durchgeführt wird.

4. Verfahren gemäss Patentanspruch 1, dadurch gekennzeichnet, dass die Quatärnisierung des N,N-disubstituierten Undeka-2Z,5Z-dienylamins durch Zugabe eines Alkylhalogenids, gefolgt von einer Behandlung mit Kalium-tert.-butylat, oder durch Methylierung und gleichzeitige Eliminierung mit Dimethylsulfat in Anwesenheit von Natriumhydroxid unter Rückfluss durchgeführt wird.

5. Ein N,N-disubstituiertes Undeka-2,5-diinylamin der Formel
H₁₁C₅-C≡C-CH₂-C≡C-CH₂NR₂
worin R für einen niederen Alkylrest steht.

6. N,N-Diethyl-undeka-2,5-diinylamin.

7. Verfahren zur Herstellung von Undeka-1,3E,5Z-trien, dadurch gekennzeichnet, dass man:
i. Deka-1,4-diin mit einem Alkylmagnesiumhalogenid, danach mit Formaldehyd umsetzt, um Undeka-2,5-diin-1-ol zu erhalten,
ii. das obengenannte Diinol einer katalytischen Hydrierung unterwirft, um Undeka-2Z,5Z-dienol zu erhalten,
iii. das so gebildete Dienol in das Undeka-2Z,5Z-dienylchlorid überführt und
iv. die letztgenannte Verbindung mit Hilfe eines basischen Mittels, bestehend aus einem Alkalimetallalkoholat, einer Eliminierungsreaktion unterwirft.

8. Verfahren gemäss Patentanspruch 7, dadurch gekennzeichnet, dass man als Alkalimetallalkoholat Kalium-tert.-butylat verwendet.
